# EUROPEAN PATENT SPECIFICATION

(11) **EP 0 641 366 B1**
(45) Date of publication and mention of the grant of the patent: **28.07.1999**
(21) Application number: 93911683.6
(22) Date of filing: 04.05.1993
(51) Int. Cl.: C07C 69/82, C07C 63/26

(54) **IMPROVED POLY ETHYLENE TEREPHTHALATE DECONTAMINATION**
SÄUBERUNG VON POLYETHYLENE TEREPHTHALATEN
AMELIORATION APPORTEE A LA DECONTAMINATION DE TEREPHTALATE DE POLYETHYLENE (PET)

(30) Priority: 18.05.1992 AU PL247092; 27.01.1993 AU PL695193
(43) Date of publication of application: 08.03.1995
(73) Proprietor: Swig Pty. Ltd., Malvern, Victoria 3144 (AU)
(72) Inventor: WEST, Simon, Michael, Williamstown, VIC 3016 (AU)
(74) Representative: Wibbelmann, Jobst, Dr., Dipl.-Chem.
(86) International application number: AU9300198
(87) International publication number: WO9323465

(56) References cited:
- DE-B- 1 247 291
- FR-A- 1 081 681
- GB-A- 610 136
- GB-A- 1 061 542
- GB-A- 1 334 558
- GB-A- 2 123 403
- US-A- 3 257 335
- US-A- 3 544 622
- US-A- 4 078 143

## Description

The invention relates a method for the decontamination of poly ethylene terephthalate ("PET").

PET is a thermoplastic polyester that can be formed from 1,2-dihydroxyethane ("ethanediol") and terephthalic acid by direct esterification to form bis (hydroxy ethyl) terephthalate ester ("BHT") which is then polymerised by catalysed ester exchange to useful polymers.

Traditionally, PET has been used extensively because it can be offered as an oriented film or fibre, has high tenacity, good electrical resistance and low moisture absorption together with a melting point of approximately 265 degrees Celsius.

For these reasons, its uses have been very diverse extending from being blended with cotton for wash and wear fabrics, blended with wool for worsteds and suitings, packaging films and recording tapes and containers including soft drink containers.

There are a number of applications of PET where remelting and reforming is not permissible or gives inferior properties. The reuse of PET for these applications is best achieved by degrading the polymer into the original monomers namely ethanediol and terephthalic acid then reacting the monomers together to regenerate the original PET.

The known art (British Patent No. 610135) is to hydrolyse PET with either strong alkalis or acids. The alkalis must be neutralised with acid to produce terephthalic acid resulting in a significant cost of reagents and possible contamination of the product with alkali metal ions. The strong acids must be recovered and the ethanediol separated. Further the acids dissolve paper and pigments to give by products which are difficult to separate.

It is known that PET will transesterify with ethanediol (British Patent No. 610136) but the product BHT, is not a convenient intermediate for subsequent purification to remove esters of contaminant acids such as benzoic and adipic acid.

Accordingly, the present invention is directed to an improved process of decontaminating PET.

### DESCRIPTION OF THE INVENTION

Accordingly, in one form of this invention, there is provided a process for removing contaminants from PET components by depolymerising PET comprising the following steps:
(a) transesterifying material containing PET by reaction with ethanediol having a temperature at or about the boiling point of ethanediol for a period of time sufficient to form a mixture containing embrittled PET; and
(b) crushing the mixture and separating crushed material containing PET from uncrushed material.

Further embodiments of the invention are set forth in the set of claims attached to the specification.

According to one aspect of the present invention, there is provided a process for removing contaminants from crushed material containing PET obtainable by the process defined in claims, which comprises:
(a) transesterifying the crushed material containing PET by reaction with ethanediol for a period of time sufficient to form a solution containing soluble short chain PET polymers and/or bis (hydroxyethyl) terephthalate ester (BHT); and
(b) recovering short chain PET polymers and/or BHT and ethanediol.

Another embodiment of the invention relates to a process for removing contaminants from crushed material containing PET obtainable by the process defined in claim 1, which comprises:
(a) hydrolysing the crushed material containing PET at elevated pressure and temperature for a period of time sufficient to form an ethanediol solution and crystals of terephthalic acid;
(b) esterifying the ethanediol solution and crystals of terephthalic acid to short chain PET polymers and/or bis(hydroxyethyl) terephthalate ester (BHT); and
(c) recovering short chain PET polymers and/or BHT.

It has been surprisingly found that ethanediol at or near its boiling point reacts with PET but does not react significantly with products often used in PET containing products including paper, other plastics (for example, poly vinyl chloride ("PVC"), colouring dyes, pigments, mineral sands or clays ("Contaminants").

Typically the step or steps involving transesterification with ethanediol are carried out at a temperature of within 10 degrees Celsius of the boiling point of ethanediol and more preferably at a temperature of within 5 degrees Celsius of the boiling point of ethanediol. Such temperature fluctuations do not substantially affect the lack of reactivity of the ethanediol with the Contaminants.

Transesterificatlon of PET with ethanediol according to the invention can result in the PET either becoming embrittled or solubilising depending on the period of time over which the transesterification is allowed to take place. At the various stages, separation of the PET containing mixtures or solutions can be affected by known separation techniques.

Preferably, embrittlement is caused to occur after a period of between between 20 minutes and 60 minutes and more preferably between 30 minutes and 50 minutes.

In the case of solubilisation, the transesterification takes place over n preferred period of at least one or two hours and even more preferably about two hours. PET will solubilise after longer periods of transesterification because of the solubility of short chain PET polymers and/or BHT in ethanediol. If all or a substantial portion of the PET is transesterified to BHT which raises the boiling point of the solution, the reaction time may be less because the higher boiling temperature will drive the reaction more quickly.

Embrittlement which takes place prior to the collapse of the original PET structure allows the PET to be crushed to separate it from Contaminants. This allows separation to take place on the basis of size and density. Crushing to less than 1 millimetre is preferred. Crushing can be achieved by rollers or hammer mills or any other known technique for reducing the size of particles. Once crushed, the PET is separated from typical Contaminants such as hydrocarbons and pigments from paper. Other substances giving rise to coloured products can be separated by screening, washing or density separation from other Contaminants which have not become embrittled or have solubilised in the ethanediol such as paper fragments in ethanediol or pulp in water medium.

Once solubilised, separation of the PET rich fraction in one form of the invention can take place preferably by filtration methods and more preferably by high pressure filtration methods. The addition of activated carbon or a combination of activated carbon and activated clay is used as an adsorbant for a wide variety of molecules such as dyes, pesticides, coloured polymers, etc.

Other foreign plastics with an alkane chain do not depolymerise in the boiling ethanediol but only melt so that they may be removed by either flotation on the denser ethanediol (1114 kilograms/m³), screened or filtered from embrittled PET or the short chain PET polymer and/or BHT solution. Proteins and polyamides do not react with ethanediol and are recovered with the other plastics. Other polyesters are also transesterified and contaminate the short chain PET polymer solution. it can be understood that this present invention does not claim that all the PET is converted to BHT only that the polymer chain length is short so that the terephthalate containing molecules are soluble in hot ethanediol to permit separation of foreign materials and rapid subsequent hydrolysis.

Following transesterification, the short chain PET polymers and/or BHT can be purified by crystallisation in water liberating excess ethanediol. The precipitate is filtered off. The filtrate containing ethanediol is recovered by fractional distillation from the additional water (British Patent No 610136). Water soluble impurities such as sugar and citric acid from soft drinks are partially removed in the filtrate and rejected as an involatile residue by distilling off the ethanediol.

Although it will be understood that the proportions of ethanediol to PET is not critical to the transesterification of the PET, in preferred forms of the invention the proportion of ethanediol to PET is at least 1:1. Excess ethanediol can be recovered by distillation techniques well known to those skilled in the art.

The short chain PET polymers and/or BHT are hydrolysed with water at elevated pressures and temperatures to give ethanediol and crystalline terephthalic acid. Typically, hydrolysis is achieved by dispersing the short chain PET polymers and/or BHT in a water slurry in a high pressure vessel and heating it to more than 180 degrees Celsius for 2 hours and then allowing it to cool. This treatment gives good hydrolysis without the requirement for catalysts (which contaminate the product) and also gives good crystals of terephthalic acid with a high purity due to the greatly increased solubility at the higher temperature. The crystals are washed with water to remove ethanediol which is recovered as described before.

A number of water soluble substances (for example, citric acid, phosphoric acid, sodium chloride and sulphuric acid) in the feed stock will be present in the mixture of terephthalic acid and ethanediol and are separated by the filtration. Additionally, a wide variety of compounds such as protein, paper, fats and some pesticides will be hydrolysed and made water soluble relative to the very insoluble terephthalic acid.

Contamination of the short chain PET polymers and/or BHT by benzoic, adipic and sebacic acid is rectified because the free acids are approximately 1000 times more soluble in the wash water allowing some foreign polyester in the feed.

The hydrolysis can be improved by using a reactor of annular format where heating is followed by partial cooling to allow the solubility of terephthalic acid to cycle and assist crystallisation and removal from the aqueous phase. Further improvement in hydrolysis is given by allowing some boiling of the hydrolysing mixture then condensing and removing an ethanediol rich phase. The contaminant water soluble substances can be removed either prior to the transesterification by water washing and drying or after hydrolysis in the water and ethanediol solution and then are rejected in the residue after distillation of the water and ethanediol.

The ethanediol is readily purified by distillation and may be partially recycled to give both product for further polymerisation and enough product to react with fresh PET. Contaminant alcohols such as methanol from ethylene methyl acrylate or hexanediol from polyester are separated in the distillation step.

The invention also provides a process for recycling PET from sources of used PET, which process comprises the processes described above. The invention further provides a process for recycling PET using diols other than ethanediol using the processes described above.

One form of the invention is illustrated in the flow chart.

Used PET is initially subjected to transesterification with boiling ethanediol for sufficient time to embrittle PET. Thereafter the mixture is passed through rollers which crush embrittled PET.

The non-crushed (ductile) components of the mixture are separated by screening from the remainder of the mixture. The PET fines are then subjected to a further transesterification process.

Activated carbon and clay is added during transesterification to adsorb contaminants. During this further transesterification process the PET is converted into short chain PET polymers and/or BHT which are soluble.

The resultant solution is subjected to a further separation by screening and/or high pressure filtration to remove contaminants such as activated carbon, clay, dye, PVC and glue.

The filtrate is hydrolysed with very pure water under elevated pressures and temperatures to form ethanediol and crystalline terephthalic acid. Acids (for example, benzoic and citric acids) remain in solution and are separated from crystalline terephthalic acid.

The ethanediol and terephthalic acid are esterified to short chain PET polymers and/or BHT. Activated carbon and activated clay are added and then any remaining contaminants for example PVC, glue and dye are removed by filtration.

### EXAMPLES

The invention is illustrated by the following non-limiting examples.

### Example 1

1 kilogram of ethanediol was heated to 190 degrees Celsius then 600 grams of granulated PET soft drink bottle was added and heated for 45 minutes. The solid PET was screened off then crushed between rolls to particles less than 1 millimetre, while paper label passed through the rolls unaffected. The crushed PET was divided into equal parts which were treated separately. One part was screened using ethanediol as an aid then the fines passing the screen were heated for one hour to convert the PET to soluble products. A mixture of 5 grams of activated carbon and 5 grams of activated clay were added and the mixture heated and stirred for 15 minutes then filtered and the product hydrolysed to give short chain PET polymers and/or BHT and ethanediol. The second part was screened with water then the product PET purified by washing with hindered settling to remove fine paper fibres and small particles. The product was dried and treated as above.

### Example 2

A reactor was constructed from 3 metres of 21 millimetre pipe and a 1 litre bowl as an annulus with the plane of the ring held vertical. 1.5 litres of water was added to the reactor and the temperature brought to 200 degrees Celsius then a molten mixture of 100 grams of short chain PET polymers and/or BHT and 100 grams of ethanediol added to the water and maintained at 200 degrees Celsius for two hours by heating one segment of the annulus and allowing the rest to be partially cooled by the atmosphere. The apparatus was then cooled and crystalline terephthalic acid and ethanediol recovered from the water suspension.

### Example 3

A sample of terephthalic acid (100 grams) was taken and dispersed in 1 litre of ethanediol and heated at the boiling point with reflux to remove water of reaction for 4 hours. A mixture of 2 grams of activated carbon and 2 grams of activated clay was added and stirred for 30 minutes then filtered off to remove PVC, PET dyes etc and then the excess ethanediol was distilled off and the polymerisation was completed by heating to 270 degrees Celsius under vacuum to produce a PET polymer.

The process described above can be used to effectively treat a variety of materials which contain PET to give pure monomers for repolymerisation. This discovery allows the use of dirty whole bottles with unsorted foreign plastics and attached labels and tops with some foreign bottles and plastic debris. The process can also be used to recover PET monomers from used X-ray film or mixed fabrics.

Since modifications to the steps described are various and obvious to those skilled in this art it is to be understood that this invention is not limited to the particular embodiments described.

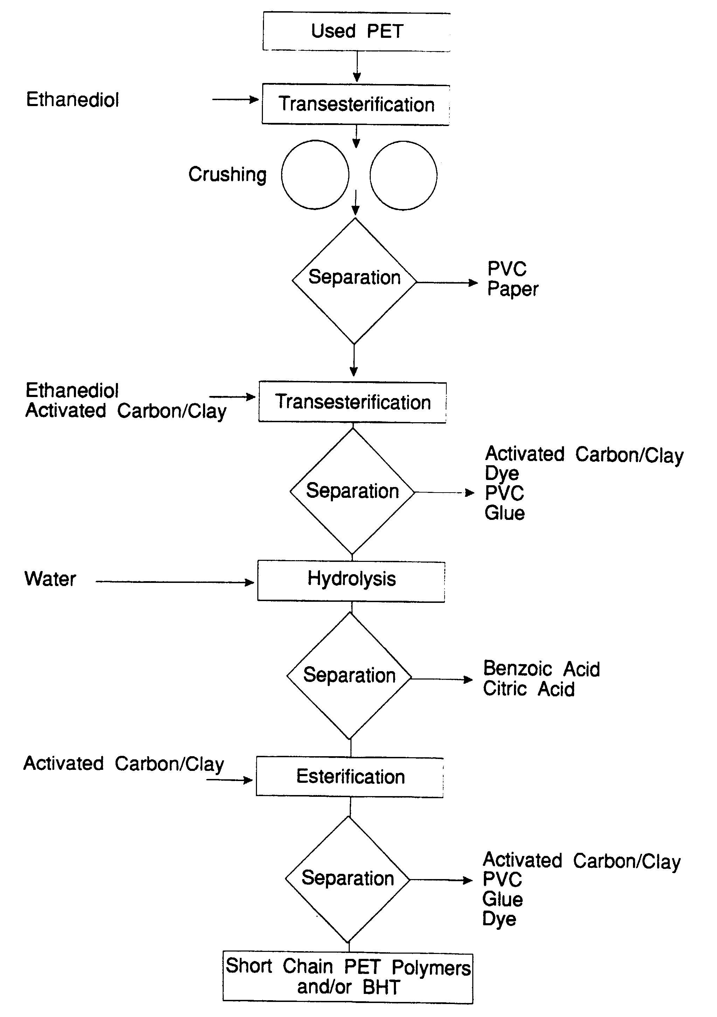

## Claims

1. A process for removing contaminants from material containing polyethylene terephthalate (PET), which comprises:
(a) transesterifying material containing PET by reaction with ethanediol having a temperature at or about the boiling point of ethanediol for a period of time sufficient to form a mixture containing embrittled PET; and
(b) crushing the mixture and separating crushed material containing PET from uncrushed material.

2. A process according to claim 1 further comprising:
(c) transesterifying the crushed material containing PET obtained in (b) by reaction with ethanediol having a temperature at or about the boiling point of ethanediol for a period of time sufficient to form a solution containing soluble short chain PET polymers and/or bis(hydroxyethyl) terephthalate ester (BHT); and
(d) recovering short chain PET polymers and/or BHT and ethanediol.

3. A process according to claim 2, further comprising:
(e) hydrolysing the recovered short chain PET polymers and/or BHT at elevated pressure and temperature for a period of time sufficient to form an ethanediol solution and crystals of terephthalic acid.

4. A process according to claim 1, further comprising:
(c) hydrolysing the crushed material containing PET obtained in (b) at elevated pressure and temperature for a period of time sufficient to form an ethanediol solution and crystals of terephthalic acid;
(d) esterifying the ethanediol solution and crystals of terephthalic acid to short chain PET polymers and/or bis(hydroxyethyl) terephthalate ester (BHT); and
(e) recovering short chain PET polymers and/or BHT.

5. A process for removing contaminants from crushed material containing PET obtainable by the process defined in claim 1, which comprises:
(a) transesterifying the crushed material containing PET by reaction with ethanediol for a period of time sufficient to form a solution containing soluble short chain PET polymers and/or bis (hydroxyethyl) terephthalate ester (BHT); and
(b) recovering short chain PET polymers and/or BHT and ethanediol.

6. A process according to claim 5, further comprising:
(c) hydrolysing the recovered short chain PET polymers and/or BHT at elevated pressure and temperature for a period of time sufficient to form ethanediol and crystals of terephthalic acid.

7. A process for removing contaminants from crushed material containing PET obtainable by the process defined by claim 1, which comprises:
(a) hydrolysing the crushed material containing PET at elevated pressure and temperature for a period of time sufficient to form an ethanediol solution and crystals of terephthalic acid;
(b) esterifying the ethanediol solution and crystals of terephthalic acid to short chain PET polymers and/or bis(hydroxyethyl) terephthalate ester (BHT); and
(c) recovering short chain PET polymers and/or BHT.

8. A process according to any one of claims 1 to 6, wherein transesterification is carried out at a temperature of within 10°C of the boiling point of ethanediol.

9. A process according to claim 8, wherein transesterification is carried out at a temperature of within 5°C of the boiling point of ethanediol.

10. A process according to any one of claims 1 to 6, 8 or 9, wherein in the transesterification the proportion of ethanediol to PET is at least 1:1.

11. A process according to claim 2 or 5, wherein the period of time sufficient to form a solution containing soluble short chain PET polymers and/or BHT is at least one hour.

12. A process according to claim 11, wherein the period of time is at least two hours.

13. A process according to claim 2 or 5, wherein the period of time sufficient to form a solution containing soluble short chain PET polymers and/or BHT is about two hours.

14. A process according to any one of claims 1 to 4, wherein the period of time sufficient to form a mixture containing embrittled PET is between 20 and 60 minutes.

15. A process according to claim 14, wherein the period of time is between 30 and 50 minutes.

16. A process according to any one of claims 1 to 4, wherein the mixture containing embrittled PET is crushed to a size less than 1 millimetre.

17. A process according to any one of claims 1 to 4, wherein separation of the crushed material containing PET from uncrushed material includes density separation.

18. A process according to any one of claims 2 to 7, wherein recovery of the PET and/or BHT and ethanediol is by filtration.

19. A process according to claim 18, wherein the filtration is high pressure filtration.

20. A process according to claim 18 or 19, wherein filtration includes the addition of activated carbon or a combination of activated carbon and activated clay.

21. A process according to claim 20, wherein activated carbon or a combination of activated carbon and activated clay is added to adsorb dyes, pesticides and/or coloured polymers.

22. A process according to claim 20, wherein activated carbon or a combination of activated carbon and activated clay is added to decolourise the PET and/or BHT and ethanediol.

23. A process for recycling PET from sources of used PET comprising the processes of any one of claims 1 to 22.

24. A process for recycling PET using diols other than ethanediol which comprises the processes of any one of claims 1 to 22.

## Patentansprüche

1. Verfahren zur Entfernung von Kontaminanten aus Polyethylenterephthalat (PET) enthaltendem Material, das folgendes umfaßt:
(a) Umestern von PET enthaltendem Material durch Umsetzen mit Ethandiol mit einer Temperatur von oder von etwa dem Siedepunkt von Ethandiol während einer ausreichenden Zeitdauer, um ein Gemisch, das versprödetes PET enthält, zu bilden; und
(b) Zerkleinern des Gemischs und Abtrennen des zerkleinerten PET enthaltenden Materials von unzerkleinertem Material.

2. Verfahren gemäß Anspruch 1, ferner umfassend:
(c) Umestern des in (b) erhaltenen zerkleinerten PET enthaltenden Materials durch Umsetzung mit Ethandiol bei einer Temperatur von oder von etwa dem Siedepunkt von Ethandiol während einer ausreichenden Zeitdauer, um eine Lösung zu bilden, die lösliche kurzkettige PET-Polymere und/oder Bis(hydroxyethyl)terephthalatester (BHT) enthält; und
(d) Gewinnen von kurzkettigen PET-Polymeren und/oder BHT und Ethandiol.

3. Verfahren gemäß Anspruch 2, ferner umfassend:
(e) Hydrolysieren der gewonnenen kurzkettigen PET-Polymere und/oder BHT bei erhöhtem Druck und erhöhter Temperatur während einer ausreichenden Zeitdauer, um eine Ethandiollösung und Kristalle von Terephthalsäure zu bilden.

4. Verfahren gemäß Anspruch 1, ferner umfassend:
(c) Hydrolysieren des in (b) erhaltenen zerkleinerten PET-haltigen Materials bei erhöhtem Druck und erhöhter Temperatur während einer ausreichenden Zeitdauer, um eine Ethandiollösung und Kristalle von Terephthalsäure zu bilden;
(d) Umestern der Ethandiollösung und der Kristalle von Terephthalsäure zu kurzkettigen PET-Polymeren und/oder Bis(hydroxyethyl)terephthalatester (BHT); und
(e) Gewinnen von kurzkettigen PET-Polymeren und/oder BHT.

5. Verfahren zum Entfernen von Kontaminanten aus durch das in Anspruch 1 definierte Verfahren erhältlichem PET-haltigem zerkleinertem Material, das folgendes umfaßt:
(a) Umestern des zerkleinerten PET enthaltenden Materials durch Umsetzen mit Ethandiol während einer ausreichenden Zeitdauer, um eine Lösung zu bilden, die lösliche kurzkettige PET-Polymere und/oder Bis(hydroxyethyl)terephthalatester (BHT) enthält; und
(b) Gewinnen von kurzkettigen PET-Polymeren und/oder BHT und Ethandiol.

6. Verfahren gemäß Anspruch 5, ferner umfassend:
(c) Hydrolysieren der gewonnenen kurzkettigen PET-Polymere und/oder BHT bei erhöhtem Druck und erhöhter Temperatur während einer ausreichenden Zeitdauer, um Ethandiol und Kristalle aus Terephthalsäure zu bilden.

7. Verfahren zum Entfernen von Kontaminanten aus durch das von Anspruch 1 definierte Verfahren erhältlichem PET-haltigem zerkleinertem Material, das folgendes umfaßt:
(a) Hydrolysieren des zerkleinerten PET-haltigen Materials bei erhöhtem Druck und erhöhter Temperatur während einer ausreichenden Zeitdauer, um eine Ethandiollösung und Kristalle aus Terephthalsäure zu bilden;
(b) Verestern der Ethandiollösung und der Kristalle aus Terephthalsäure zu kurzkettigen PET-Polymeren und/oder Bis(hydroxyethyl)terephthalatester (BHT); und
(c) Gewinnen von kurzkettigen PET-Polymeren und/oder BHT.

8. Verfahren gemäß mindestens einem der Ansprüche 1 bis 6, wobei die Umesterung bei einer Temperatur innerhalb von 10°C vom Siedepunkt von Ethandiol durchgeführt wird.

9. Verfahren gemäß Anspruch 8, wobei die Umesterung bei einer Temperatur innerhalb von 5°C des Siedepunktes von Ethandiol durchgeführt wird.

10. Verfahren gemäß mindestens einem der Ansprüche 1 bis 6, 8 oder 9, wobei bei der Umesterung das Verhältnis von Ethandiol zu PET mindestens 1:1 beträgt.

11. Verfahren nach Anspruch 2 oder 5, wobei die zur Bildung einer Lösung, die lösliche kurzkettige PET-Polymere und/oder BHT enthält, ausreichende Zeitdauer mindestens eine Stunde beträgt.

12. Verfahren gemäß Anspruch 11, wobei sich die Zeitdauer auf mindestens zwei Stunden beläuft.

13. Verfahren gemäß Anspruch 2 oder 5, wobei die zur Bildung einer Lösung, die lösliche kurzkettige PET-Polymere und/oder BHT enthält, ausreichende Zeitdauer etwa zwei Stunden beträgt.

14. Verfahren gemäß mindestens einem der Ansprüche 1 bis 4, wobei die zur Bildung eines Gemisches, das versprödetes PET enthält, ausreichende Zeitdauer zwischen 20 und 60 Minuten beträgt.

15. Verfahren gemäß Anspruch 14, wobei die Zeitdauer zwischen 30 und 50 Minuten liegt.

16. Verfahren gemäß mindestens einem der Ansprüche 1 bis 4, wobei das Gemisch, das versprödetes PET enthält, zu einer Größe von weniger als 1 Millimeter zerkleinert wird.

17. Verfahren gemäß mindestens einem der Ansprüche 1 bis 4, wobei die Abtrennung des zerkleinerten PET-haltigen Materials von unzerkleinertem Material eine Dichte-Separation einschließt.

18. Verfahren gemäß mindestens einem der Ansprüche 2 bis 7, wobei das Gewinnen von dem PET und/oder von BHT und Ethandiol durch Filtration erfolgt.

19. Verfahren gemäß Anspruch 18, wobei die Filtration eine Hochdruckfiltration ist.

20. Verfahren gemäß Anspruch 18 oder 19, wobei die Filtration die Zugabe von Aktivkohle oder einer Kombination von Aktivkohle und aktivierten Ton einschließt.

21. Verfahren gemäß Anspruch 20, wobei Aktivkohle oder eine Kombination von Aktivkohle und aktiviertem Ton zugegeben wird, um Farbstoffe, Pestizide und/oder gefärbte Polymere zu adsorbieren.

22. Verfahren gemäß Anspruch 20, wobei Aktivkohle oder eine Kombination von Aktivkohle und aktiviertem Ton zugegeben wird, um das PET und/oder BHT und Ethandiol zu entfärben.

23. Verfahren für das Recycling von PET aus Quellen von gebrauchtem PET, das die Verfahren von mindestens einem der Ansprüche 1 bis 22 umfaßt.

24. Verfahren für das Recycling von PET unter Verwendung von Diolen, die von Ethandiol verschieden sind, welches die Verfahren von mindestens einem der Ansprüche 1 bis 22 umfaßt.

## Revendications

1. Procédé pour enlever des contaminants d'un matériau contenant du téréphtalate de polyéthylène (PET), qui comprend les opérations consistant à :
(a) transestérifier un matériau contenant du PET par la réaction avec de l'éthanediol ayant une température égale au ou proche du point d'ébullition de l'éthanediol pendant une période suffisante pour former un mélange contenant du PET rendu cassant; et
(b) broyer le mélange et séparer le matériau broyé contenant du PET du matériau non broyé.

2. Procédé selon la revendication 1 comprenant en outre les opérations consistant à :
(c) transestérifier le matériau broyé contenant du PET obtenu en (b) par réaction avec de l'éthanediol ayant une température égale au ou proche du point d'ébullition de l'éthanediol pendant une durée suffisante pour former une solution contenant du téréphtalate de bis(hydroxyéthyle) (BHT) et/ou des polymères de PET à chaînes courtes solubles; et
(d) récupérer le BHT et/ou les polymères de PET à chaînes courtes et l'éthanediol.

3. Procédé selon la revendication 2, comprenant en outre l'opération consistant à :
(e) hydrolyser le BHT et/ou les polymères de PET à chaînes courtes récupérés à pression et à température élevées pendant une durée suffisante pour former une solution d'éthanediol et des cristaux d'acide téréphtalique.

4. Procédé selon la revendication 1, comprenant en outre les opérations consistant à :
(c) hydrolyser le matériau broyé contenant du PET obtenu en (b) à pression et à température élevées pendant une durée suffisante pour former une solution d'éthanediol et des cristaux d'acide téréphtalique;
(d) estérifier la solution d'éthanediol et les cristaux d'acide téréphtalique en téréphtalate de bis(hydroxyéthyle) (BHT) et/ou polymères de PET à chaînes courtes; et
(e) récupérer le BHT et/ou les polymères de PET à chaînes courtes.

5. Procédé pour enlever des contaminants d'un matériau broyé contenant du PET pouvant être obtenu par le procédé défini dans la revendication 1, comprenant les opérations consistant à :
(a) transestérifier le matériau broyé contenant du PET par réaction avec de l'éthanediol pendant une durée suffisante pour former une solution contenant du téréphtalate de bis(hydroxyéthyle) (BHT) et/ou des polymères de PET à chaînes courtes solubles; et
(b) récupérer les polymères à chaîne courte de PET et/ou le BHT et l'éthanediol.

6. Procédé selon la revendication 5, comprenant en outre l'opération consistant à :
(c) hydrolyser le BHT et/ou les polymères de PET à chaînes courtes récupérés à pression et à température élevées pendant une durée suffisante pour former de l'éthanediol et des cristaux d'acide téréphtalique.

7. Procédé pour enlever des contaminants d'un matériau broyé contenant du PET pouvant être obtenu par le procédé défini par la revendication 1, comprenant les opérations consistant à :
(a) hydrolyser le matériau broyé contenant du PET à pression et à température élevées pendant une durée suffisante pour former une solution d'éthanediol et des cristaux d'acide téréphtalique;
(b) estérifier la solution d'éthanediol et les cristaux d'acide téréphtalique en téréphtalate de bis(hydroxyéthyle) (BHT) et/ou en polymères de PET à chaînes courtes; et
(c) récupérer le BHT et/ou les polymères de PET à chaînes courtes.

8. Procédé selon l'une quelconque des revendications 1 à 6, dans lequel la transestérification est effectuée à une température à moins de 10 °C du point d'ébullition de l'éthanediol.

9. Procédé selon la revendication 8, dans lequel la transestérification est effectuée à une température à moins de 5 °C du point d'ébullition de l'éthanediol.

10. Procédé selon l'une quelconque des revendications 1 à 6, 8 ou 9, dans lequel la proportion de l'éthanediol au PET est d'au moins 1:1 dans la transestérification.

11. Procédé selon la revendication 2 ou 5, dans lequel la durée suffisante pour former une solution contenant du BHT et/ou des polymères de PET à chaînes courtes solubles est d'au moins une heure.

12. Procédé selon la revendication 11, dans lequel la durée est d'au moins deux heures.

13. Procédé selon la revendication 2 ou 5, dans lequel la durée suffisante pour former une solution contenant du BHT et/ou des polymères de PET à chaînes courtes solubles est d'environ deux heures.

14. Procédé selon l'une quelconque des revendications 1 à 4, dans lequel la durée suffisante pour former un mélange contenant du PET rendu cassant se situe entre 20 et 60 minutes.

15. Procédé selon la revendication 14, dans lequel la durée se situe entre 30 et 50 minutes.

16. Procédé selon l'une quelconque des revendications 1 à 4, dans lequel le mélange contenant du PET rendu cassant est broyé à une taille de moins de 1 millimètre.

17. Procédé selon l'une quelconque des revendications 1 à 4, dans lequel la séparation du matériau broyé contenant du PET du matériau non broyé comprend une séparation par densité.

18. Procédé selon l'une quelconque des revendications 2 à 7, dans lequel la récupération du PET et/ou du BHT et de l'éthanediol s'effectue par filtration.

19. Procédé selon la revendication 18, dans lequel la filtration est une filtration à haute pression.

20. Procédé selon la revendication 18 ou 19, dans lequel la filtration comprend l'addition de charbon actif ou une combinaison de charbon actif et d'argile activée.

21. Procédé selon la revendication 20, dans lequel le charbon actif ou une combinaison de charbon actif et d'argile activée est ajouté pour adsorber des colorants, des pesticides et/ou des polymères colorés.

22. Procédé selon la revendication 20, dans lequel le charbon actif ou une combinaison de charbon actif et d'argile activée est ajouté pour décolorer le PET et/ou le BHT et l'éthanediol.

23. Procédé pour recycler le PET provenant de sources de PET usagé comprenant les procédés de l'une quelconque des revendications 1 à 22.

24. Procédé pour recycler le PET en utilisant des diols autres que l'éthanediol qui comprend les procédés de l'une quelconque des revendications 1 à 22.
